# EUROPEAN PATENT SPECIFICATION

(11) **EP 3 796 859 B1**
(45) Date of publication and mention of the grant of the patent: **16.03.2022**
(21) Application number: 19724784.4
(22) Date of filing: 14.05.2019
(51) Int. Cl.: A61B 18/18, A61B 18/14

(54) **ELECTROSURGICAL ABLATION INSTRUMENT**
ELEKTROCHIRURGISCHES ABLATIONSINSTRUMENT
INSTRUMENT D'ABLATION ÉLECTROCHIRURGICAL

(30) Priority: 19.05.2018 GB 201808165
(43) Date of publication of application: 31.03.2021
(73) Proprietor: Creo Medical Limited, Chepstow, Wales NP16 5UH (GB)
(72) Inventor: HANCOCK, Christopher Paul, Bath Bath and North East Somerset BA1 4LN (GB); SWAIN, Sandra May, Chepstow Wales NP16 5UH (GB)
(74) Representative: Mewburn Ellis LLP
(86) International application number: PCT/EP2019/062311
(87) International publication number: WO 2019/224049

(56) References cited:
- EP-A1- 2 177 173
- WO-A2-03/024309
- GB-A- 2 547 941
- US-A1- 2009 187 180

## Description

### FIELD OF THE INVENTION

The invention relates to an electrosurgical instrument for delivering radiofrequency and microwave energy to biological tissue in order to ablate the target tissue. In particular, the probe is configured to be insertable through a channel of a surgical scoping device or catheter that can be introduced to a treatment site in a non-invasive manner. The probe may be arranged to ablate tissue, such as a tumour, cyst or other lesion. The probe may be particularly suited for treatment in the pancreas.

### BACKGROUND TO THE INVENTION

The application of heat energy to biological tissue is well known as an effective method of killing cells. For example, the application of radiofrequency or microwave energy can heat and thus ablate (destroy) biological tissue. This method may in particular be used for the treatment of cancer.

A technique of treating tissue in the pancreas using endoscopic ultrasound guided radiofrequency ablation is known (Pai, M., et al.: Endoscopic ultrasound guided radiofrequency ablation, for pancreatic cystic neoplasms and neuroendocrine tumors, World J Gastrointest Surg 2015 April 27; 7(4): 52-59). In this technique a conductive wire having a small diameter (e.g. 0.33 mm) is inserted through the working channel of an ultrasound-enabled endoscope. RF power is applied to the wire in conjunction with an external grounded return pad in contact with the patient's skin to coagulate tissue in the liver and pancreas. To ablate lesions it is necessary to apply power for 90-120 seconds, and, in some cases to remove and reposition the wire.

WO 03/024309 A2 describes a tissue ablation device including a catheter shaft having an antenna lumen, an impedance-matched microwave antenna carried in the antenna lumen of the catheter shaft, at least one cooling lumen in the catheter shaft around the antenna lumen for circulation of cooling fluid, and a microwave generator operatively coupled to the antenna for energizing the antenna to create a lesion in the targeted tissue around the catheter shaft having a controlled location and size.

US 2009/0187180 A1 describes a microwave antenna assembly including a feedline having an inner conductor, an outer conductor and an inner insulator disposed therebetween.

EP 2 177 173 A1 describes a microwave antenna assembling including a feedline having an inner conductor, an outer conductor and an inner conductor disposed therebetween and a radiating section coupled to the feedline, the radiating section including a dipole antenna and a tubular dielectric loading disposed about the dipole antenna.

### SUMMARY OF THE INVENTION

At its most general, the invention provides an electrosurgical instrument having a microwave ablation antenna dimensioned to be suitable for insertion into a pancreas via a surgical scoping device, to provide a rapid and accurate alternative to known RF ablation techniques. Although the invention may find particular use in the pancreas, it may also be suitable for use in other awkward treatment sites, such as the lungs, etc. The instrument structure disclosed herein enables an ablation antenna to be provided with appropriate length and rigidity for use in a variety of setting. The length and rigidity of the ablation antenna described herein may play an important role in the final application. For very short lengths, the radiating tip portion discussed below may be dielectrically or magnetically loaded by a combination of high relative permittivity and permeability materials respectively in order to maintain the electrical integrity or performance. The length of the radiating tip portion may be in the range from 5 mm to 80 mm.

By enabling tumours within the pancreas to be ablated using a minimally invasive procedure can make viable the option of ablation treatment for both curative as well as palliative reasons.

According to the present invention, there may be provided an electrosurgical instrument as set out in claim 1.

An advantage of configuring the distal needle tip as a half wavelength transformer is to minimise reflections at the interface between components, e.g. between the coaxial cable and proximal coaxial transmission line, and between the proximal coaxial transmission line and the distal needle tip. A reflection coefficient at the latter interface is typically larger due to a larger variation in impedance. The half wavelength configuration minimises these reflections so that the dominant reflection coefficient becomes that of the interface between the proximal coaxial transmission line and the tissue. The impedance of the proximal coaxial transmission line may be selected to be identical or close to the expected tissue impedance to provides a good match at the frequency of the microwave energy.

The radiating tip portion may comprise an intermediate coaxial transmission line between the proximal coaxial transmission line and the distal needle tip. The intermediate coaxial transmission line may be formed from an overlap between the distal needle tip and conductive components of the proximal coaxial transmission line. The intermediate coaxial transmission line may be provided with a higher dielectric constant than the proximal coaxial transmission line to allow for a smaller physical length whilst getting the required electrical length (half wave). A distal portion of the distal needle tip acts as an open-ended loaded monopole connected to the intermediate coaxial transmission line. The distal needle tip may also be considered as a single structure which ends in an open-ended co-axial monopole to shape the ablation zone.

The proximal coaxial transmission line may comprise: an inner conductor that extends from a distal end of the flexible coaxial cable, the inner conductor being electrically connected to an centre conductor of the flexible coaxial cable; a proximal dielectric sleeve mounted around the inner conductor; and a outer conductor mounted around the proximal dielectric, wherein the distal needle tip comprises a distal dielectric sleeve mounted around the inner conductor, and wherein a distal portion of the outer conductor overlays a proximal portion of the distal dielectric sleeve. The intermediate coaxial transmission line may thus be formed by the length of distal dielectric sleeve that is overlaid by the outer conductor.

The outer conductor is a conductive tube, e.g. formed from nitinol, that exhibits longitudinal rigidity sufficient to transmit a force capable of penetrating the duodenum wall. Preferably the conductive tube also exhibits lateral flex suitable to enable the instrument to travel through the instrument channel of a surgical scoping device. The radiating tip portion is substantially rigid to permit insertion into biological tissue.

The inner conductor may be formed from a material with high conductivity, e.g. silver. The inner conductor may have a diameter that is less than the diameter of the centre conductor of the flexible coaxial cable. For example, the diameter of the inner conductor may be 0.25 mm. The preferred diameter takes into account that the dominant parameter that determines loss (and heating) along the radiating tip portion is the conductor loss, which is a function of the diameter of the inner conductor. Other relevant parameters are the dielectric constants of the distal and proximal dielectric sleeves, and the diameter and material used for the outer conductor.

The dimensions of the components of the proximal coaxial transmission line may be chosen to provide it with an impedance that is identical or close to the impedance of the flexible coaxial cable (e.g. around 50 Q).

The radiating tip portion may be secured to the flexible coaxial cable by a collar mounted over a junction therebetween. The collar may be electrically conductive, e.g. formed from brass. It may electrically connect the outer conductor with an outer conductor of the flexible coaxial cable.

The distal dielectric sleeve may have a bore formed therethrough for receiving the inner conductor. The distal needle tip may further comprise a tip element mounted at a distal end of the distal dielectric sleeve to close the bore. The tip element may be made from any of PEEK, epoxy, Macor, alumina, glass, glass filled PEEK.

A distal end of the distal dielectric sleeve may be sharpened, e.g. may taper to a point. This may facilitate insertion of the instrument through the duodenal or gastric wall into the pancreas.

The distal dielectric sleeve may be made from a different material to the proximal dielectric sleeve. The proximal dielectric sleeve may be made from the same material as a dielectric material of the flexible coaxial cable, e.g. PTFE or the like. In contrast, the distal dielectric sleeve may be made from any of ceramic, polyether ether ketone (PEEK), glass-filled PEEK. This materials exhibit desirable rigidity and are capable of being sharpened. It also allows for controlling (e.g. reducing or optimising) the physical length of the radiating tip portion whilst maintaining its electric length.

The radiating tip portion may have a length equal to or greater than 30 mm and preferably 40mm, but could be as long as 100 mm. This length enables access to treatment regions at all locations within the pancreas. The radiating tip portion may have a maximum outer diameter equal to or less than 1.2 mm. This may reduce or minimise the penetration hole cause by insertion of the instrument, so as not to cause an undue delay in healing. Minimising the size of the penetration hole may also avoid the undesirable situation of it healing open and causing a fistula or unwanted channel between the GI tract and the body cavity.

A microwave choke or balun may be fabricated on an outer surface of the proximal coaxial transmission line. This can assist in generating a more spherical field shape for the microwave energy emitted at the distal needle tip. A plurality of chokes may be arranged on the outer conductor. They may be applied thereon after the coaxial transmission line is formed. Each choke may have a length along the coaxial transmission line that is a quarter wavelength of the microwave energy. This is to convert a short circuit into an open circuit a quarter wavelength away. This provides isolation to prevent the propagation of surface currents back down the outer surface of the needle. The effect of this is to force energy out of the distal radiating portion. The chokes may be loaded with a dielectric material having a higher relative permittivity material to decrease physical length whilst maintaining the required electrical length (quarter wavelength).

The location of said baluns may be such that they control or help steer the ablation profile, e.g. the first balun may be placed 15 mm back from the distal radiating end of the device to ensure that the ablation profile does not extend back more than 15 mm from said location.

Also disclosed herein is an electrosurgical apparatus comprising: a surgical scoping device having an instrument cord configured to be insertable into a patient's body, wherein the instrument cord has an instrument channel formed therethrough; and an electrosurgical instrument as discussed above dimensioned to be insertable through the instrument channel.

The term "surgical scoping device" may be used herein to mean any surgical device provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube. The instrument channel may have a diameter suitable for receiving invasive surgical tools. The diameter of the instrument channel may be 5 mm or less. In embodiments of the invention, the surgical scoping device may be an ultrasound-enabled endoscope.

Herein, the term "inner" means radially closer to the centre (e.g. axis) of the instrument channel and/or coaxial cable. The term "outer" means radially further from the centre (axis) of the instrument channel and/or coaxial cable.

The term "conductive" is used herein to mean electrically conductive, unless the context dictates otherwise.

Herein, the terms "proximal" and "distal" refer to the ends of the elongate probe. In use, the proximal end is closer to a generator for providing the RF and/or microwave energy, whereas the distal end is further from the generator.

In this specification "microwave" may be used broadly to indicate a frequency range of 400 MHz to 100 GHz, but preferably the range 1 GHz to 60 GHz. Specific frequencies that have been considered are: 915 MHz, 2.45 GHz, 3.3 GHz, 5.8 GHz, 10 GHz, 14.5 GHz and 24 GHz. The device may deliver energy at more than one of these microwave frequencies.

### BRIEF DESCRIPTION OF THE DRAWINGS

Embodiments of the invention are discussed below with reference to the accompanying drawings, in which:
Fig. 1 is a schematic diagram showing an electrosurgical ablation apparatus that is an embodiment of the invention;
Fig. 2 is a schematic sectional view through an instrument cord of an endoscope that can be used with the present invention;
Fig. 3A is an exploded perspective view of an electrosurgical instrument that is an embodiment of the invention;
Fig. 3B is a perspective view of the electrosurgical instrument of Fig. 3A when assembled;
Fig. 4A is a cross-sectional view through a first example electrosurgical instrument;
Fig. 4B is a simulated power density chart for the instrument of Fig. 4A when operating at 5.8 GHz;
Fig. 4C is a simulated return loss graph for the instrument of Fig. 4A;
Fig. 5A is a cross-sectional view through a second example electrosurgical instrument;
Fig. 5B is a simulated power density chart for the instrument of Fig. 5A when operating at 5.8 GHz;
Fig. 5C is a simulated return loss graph for the instrument of Fig. 5A;
Fig. 6A is a cross-sectional view through a third example electrosurgical instrument;
Fig. 6B is a simulated power density chart for the instrument of Fig. 6A when operating at 5.8 GHz; and
Fig. 6C is a simulated return loss graph for the instrument of Fig. 6A.

### DETAILED DESCRIPTION; FURTHER OPTIONS AND PREFERENCES

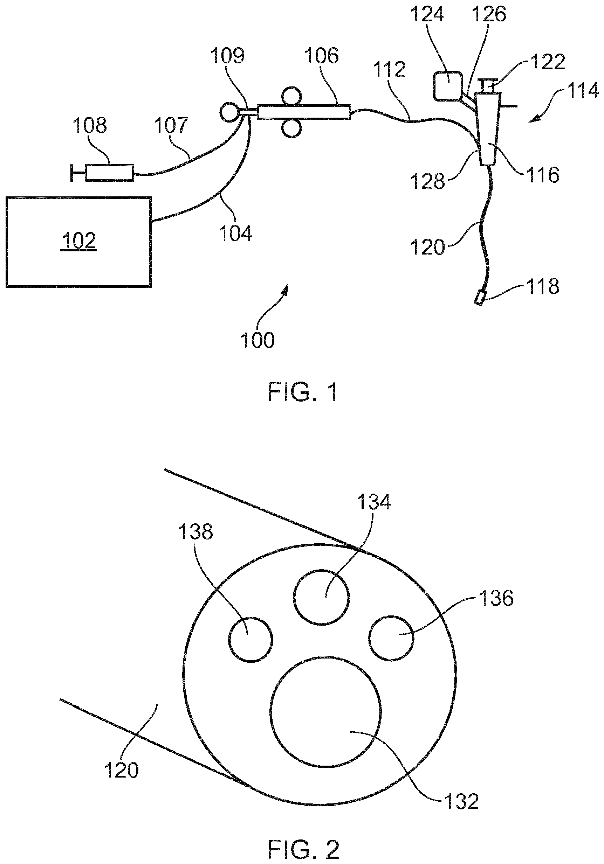

Fig. 1 is a schematic diagram of an electrosurgical ablation apparatus 100 that is capable of supplying microwave energy and fluid, e.g. cooling fluid, to the distal end of an invasive electrosurgical instrument. The system 100 comprises a generator 102 for controllably supplying radiofrequency (RF) and microwave energy. A suitable generator for this purpose is described in WO 2012/076844.

The generator may be arranged to monitor reflected signals received back from the instrument in order to determine an appropriate power level for delivery. For example, the generator may be arranged to calculate an impedance seen at the distal end of the instrument in order to determine an optimal delivery power level.

The generator 102 is connected to an interface joint 106 by an interface cable 104. The interface joint 106 is also connected via a fluid flow line 107 to a fluid delivery device 108, such as a syringe,. In some examples, the apparatus may be arranged, additionally or alternatively, to aspirate fluid from the treatment site. In this scenario, the fluid flow line 107 may convey fluid away from the interface joint 106 to a suitable collector (not shown). The aspiration mechanism may be connected at a proximal end of the fluid flow line 107.

If needed, the interface joint 106 can house an instrument control mechanism that is operable by sliding a trigger, e.g. to control longitudinal (back and forth) movement of one or more control wires or push rods (not shown). If there is a plurality of control wires, there may be multiple sliding triggers on the interface joint to provide full control. The function of the interface joint 106 is to combine the inputs from the generator 102, fluid delivery device 108 and instrument control mechanism into a single flexible shaft 112, which extends from the distal end of the interface joint 106.

The flexible shaft 112 is insertable through the entire length of an instrument (working) channel of a surgical scoping device 114, which in embodiment of the present invention may comprise an endoscopic ultrasound device.

The surgical scoping device 114 comprises a body 116 having a number of input ports and an output port from which an instrument cord 120 extends. The instrument cord 120 comprises an outer jacket which surrounds a plurality of lumens. The plurality of lumens convey various things from the body 116 to a distal end of the instrument cord 120. One of the plurality of lumens is the instrument channel discussed above. Other lumens may include a channel for conveying optical radiation, e.g. to provide illumination at the distal end or to gather images from the distal end. The body 116 may include a eye piece 122 for viewing the distal end.

An endoscopic ultrasound device typically provide an ultrasound transducer on a distal tip of the instrument cord, beyond an exit aperture of the instrument channel. Signals from the ultrasound transducer may be conveyed by a suitable cable 126 back along the instrument cord to a processor 124, which can generate images in a known manner. The instrument channel may be shaped within the instrument cord to direct an instrument exiting the instrument channel through the field of view of the ultrasound system, to provide information about the location of the instrument at the target site.

The flexible shaft 112 has a distal assembly 118 (not drawn to scale in Fig. 1) that is shaped to pass through the instrument channel of the surgical scoping device 114 and protrude (e.g. inside the patient) at the distal end of the instrument cord.

The structure of the distal assembly 118 discussed below may be particularly designed for use with an endoscopic ultrasound (EUS) device, whereby the maximum outer diameter of the distal end assembly 118 is equal to or less than 2.0 mm, e.g. less than 1.9 mm (and more preferably less than 1.5 mm) and the length of the flexible shaft can be equal to or greater than 1.2 m.

The body 116 includes a power input port 128 for connecting to the flexible shaft 112. As explained below, a proximal portion of the flexible shaft may comprise a conventional coaxial cable capable of conveying the radiofrequency and microwave energy from the generator 102 to the distal assembly 118. Coaxial cables that are physically capable of fitting down the instrument channel of an EUS device are available with the following outer diameters: 1.19 mm (0.047"), 1.35 mm (0.053"), 1.40 mm (0.055"), 1.60 mm (0.063"), 1.78 mm (0.070"). Custom-sized coaxial cables (i.e. made to order) may also be used.

As discussed above, it is desirable to be able to control the position of at least the distal end of the instrument cord 120. The body 116 may include a control actuator that is mechanically coupled to the distal end of the instrument cord 120 by one or more control wires (not shown), which extend through the instrument cord 120. The control wires may travel within the instrument channel or within their own dedicated channels. The control actuator may be a lever or rotatable knob, or any other known catheter manipulation device. The manipulation of the instrument cord 120 may be software-assisted, e.g. using a virtual three-dimensional map assembled from computer tomography (CT) images.

Fig. 2 is a view down the axis of the instrument cord 120. In this embodiment there are four lumens within the instrument cord 120. The largest lumen is the instrument channel 132. The other lumens comprise an ultrasound signal channel 134 and an illumination channel 136, and a camera channel 138 but the invention is not limited to this configuration. For example, there may be other lumens, e.g. for control wires or fluid delivery or suction.

In one embodiment, the invention may provide an instrument that can perform tissue ablation at the distal end of an EUS system catheter. In order for side effects to be reduced and the efficiency of the instrument to be maximised, the transmitting antenna should be located as close to the target tissue as possible. Ideally, the radiating part of the instrument is located inside (e.g. at the centre of) the tumour during treatment.

The invention may be particularly suited for treatment of the pancreas. In order to reach the target site, the instrument will need to be guided through the mouth, stomach and duodenum. The instrument is arranged to access the pancreas by passing through the wall of the duodenum. This procedure places significant restrictions on the size of the instrument that may pass into the pancreas. Conventionally, instruments having an outer diameter no larger than 1 mm (e.g. 19 gauge) have been used.

The description below presents an antenna configurations that are suitable for use in the distal assembly 118 described.

In the following description, unless stated otherwise, the length of a component refers to its dimension in the direction parallel to the longitudinal axis of the coaxial cable/instrument cord.

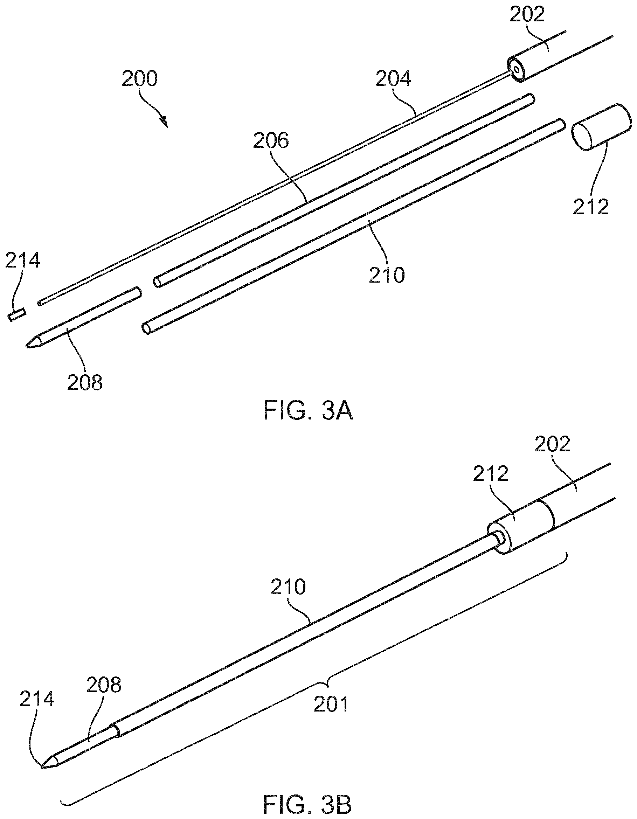

Fig. 3A is an exploded perspective view of an electrosurgical instrument 200 that is an embodiment of the invention. Fig. 3B shows the electrosurgical instrument 200 after assembly. The electrosurgical instrument comprises a coaxial cable 202 having a radiating tip structure 201 (e.g. an ablation antenna structure) mounted on a distal end thereof. The coaxial cable 202 may be a conventional flexible 50 Q coaxial cable suitable for travelling through the instrument channel of a surgical scoping device. In one example the coaxial cable 202 is a Huber and Suhner Sucoform 86 coaxial cable, but the invention is not limited to a coaxial cable of this size.

The radiating tip structure comprises a coaxial transmission line have a needle-like radiating tip mounted at its distal end. The coaxial transmission line comprise an inner conductor 204 which is electrically connected to an centre conductor (not shown) of the coaxial cable 202. In some examples, the inner conductor 204 may be a continuation of the centre conductor of the coaxial cable 202, i.e. a portion thereof that protrudes from a distal end of the cable. However, typically the outer diameter of such conductors is too large to be suitable for use in the present invention, so a separate thinner conductor may be used. The inner conductor 204 may be formed from silver or other highly conductive material.

The inner conductor 204 is surrounded along a proximal portion thereof (which may correspond to the coaxial transmission line) by a proximal dielectric sleeve 206, which may be a tube or PTFE or the like. The proximal dielectric sleeve 206 terminates before a distal end of the inner conductor 204. A distal dielectric sleeve 208 is mounted over a distal portion of the inner conductor 204 to form the radiating tip. The distal dielectric sleeve 208 may be formed from a hard insulating material that can be sharpened at its distal end to be suitable for insertion into biological tissue. For example, the distal dielectric sleeve 208 may be a ceramic (e.g. alumina), polyether ether ketone (PEEK), or a blend thereof, e.g. glass-filled PEEK.

The coaxial transmission line is completed by a outer conductor 210 mounted around the proximal dielectric sleeve 206. The outer conductor 210 is preferably a rigid tube, e.g. of metal or other suitable conductive material. The tube is configured to have longitudinal rigidity sufficient to transmit a force capable of penetrating the duodenum wall, whilst also exhibiting suitable lateral flex to enable the instrument to travel through the instrument channel of a surgical scoping device. It has been found that nitinol exhibit appropriate behaviour, but other materials may also be used, e.g. stainless steel or the like. The length of the outer conductor 210 is chosen so that is extends beyond a distal end of the proximal dielectric sleeve 206. In other words, the junction between the proximal dielectric sleeve 206 and the distal dielectric sleeve 208 is located within the outer conductor 210. There is therefore an intermediate coaxial transmission line formed by the inner conductor 204, distal dielectric sleeve 208 and outer conductor 210 in a region proximal to the exposed needle-like distal dielectric sleeve 208. The length of the intermediate coaxial transmission line may be selected to improve the impedance match along the instrument.

The proximal dielectric sleeve 206 and the distal dielectric sleeve 208 may be formed as tubes that slide over the inner conductor 204. At the distalmost end of the instrument, an insulating tip element 214 may be mounted to close a distal end of the distal dielectric sleeve 208, e.g. to close a bore formed therein for receiving the inner conductor 204. The tip element 214 and/or the distal end of the distal dielectric sleeve 208 may be sharpened, e.g. to form a needle-like structure for insertion into biological tissue. The tip element 214 may be made from the same material as the distal dielectric sleeve 208, e.g. PEEK or the like. However, other insulating materials may be used, e.g. epoxy, Macor, alumina, glass, glass filled PEEK, etc.

The radiating tip portion 201 is secured to the distal end of the coaxial cable 202 by a collar 212. The collar 212 may act as a radial crimp to secure the radiating tip portion 201 in place. The collar 212 is also arranged to electrically connect the outer conductor of the coaxial cable to the outer conductor 210 of the coaxial transmission line. The collar 212 is thus formed from a conductive material, e.g. brass or the like.

The electrosurgical instrument 200 is configured for use as an ablation antenna to emit microwave energy received along the coaxial cable into biological tissue. The electrosurgical instrument is designed in particular to be suitable for insertion through an instrument channel of a surgical scoping device (e.g. an endoscopic ultrasound (EUS) apparatus) to a treatment site. The treatment site may be the pancreas, whereby an instrument cord of the surgical scoping device is inserted into the duodenum, whereupon the electrosurgical instrument 200 is extended to penetrate through the wall of the duodenum into the pancreas to treatment.

The electrosurgical instrument may have several features that render it suitable for use in this context. The rigid portion of the instrument desirably has a length equal to or greater than 40 mm with a maximum outer diameter of 1.2 mm. This can ensure the needle is long enough to reach tumours located within the pancreas, and can ensure that the penetration hole is not too large, which can delay healing.

In one example, assembly of the instrument comprises drilling a 0.3 mm hole into the centre conductor of the coaxial cable 202. The inner conductor 204 (which may be a needle element having an outer diameter of 0.25 mm) is then inserted into th hole and secured with solder. The proximal dielectric sleeve 206 (e.g. PTFE tubing) is pushed over the inner conductor 204 taking care to eliminate any air gaps at the connection. The distal dielectric sleeve 208 (e.g. PEEK section) is then pushed onto the inner conductor 204 and secured with a small amount of medical grade epoxy. The outer conductor 210 (e.g. nitinol tube) is then be pushed over the distal dielectric sleeve and proximal dielectric sleeve. The collar 212 is placed over the junction between the outer conductor 210 and coaxial cable 202 and secured with solder. Finally the tip element (e.g. PEEK) is inserted into the distal dielectric sleeve and secured with a small amount of epoxy. Table 1 lists the component dimensions in this example.

**Table 1: Component dimensions**

| **Component** | **Length (mm)** | **Outer diameter (mm)** |
|---|---|---|
| Tip 214 | 1.5 | 0.8 |
| Inner conductor 204 | 47 | 0.25 |
| Coaxial cable 202 | 1500 | 2.1 |
| Distal dielectric 208 | 11 | 0.8 |
| Proximal dielectric 206 | 37 | 0.8 |
| Outer conductor 210 | 41 | 1.01 |
| Collar 212 | 4 | 2.3 |

CST Microwave Studio was used to design and simulate the instrument structure shown above, in three different configurations.

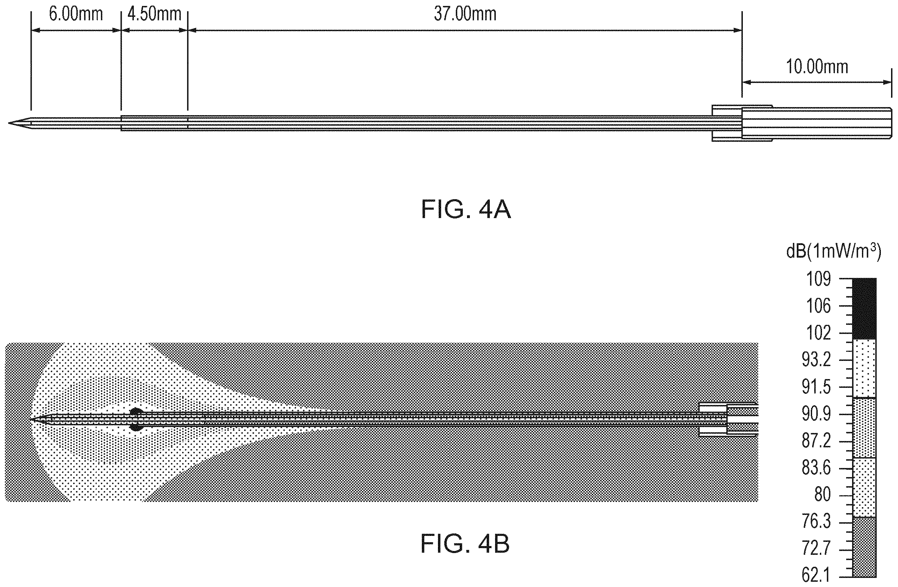

A first configuration is shown in Fig. 4A. The device can be separated into three sections: (i) a Huber and Suhner Sucoform 86 coaxial cable (50 Q impedance) which is connected to the radiating tip portion by a brass connector, (ii) a radiating tip portion (48 Q impedance) which operates as a half wavelength transformer to deliver energy into biological tissue, and (iii) the exposed sharpened distal dielectric sleeve at the distal end of the radiating tip portion is (39 Q impedance) which is terminated at its distal end in the intermediate coaxial transmission line mentioned above. In the first configuration the distal dielectric sleeve is PEEK. The intermediate coaxial transmission line adds capacitance which alters the required length of the structures from their theoretical values.

Fig. 4B shows return loss for the configuration of Fig. 4A. A match at 5.8GHz is achieved with a return loss of - 33.4dB, which equates to 99.95% of the power going into the tumour.

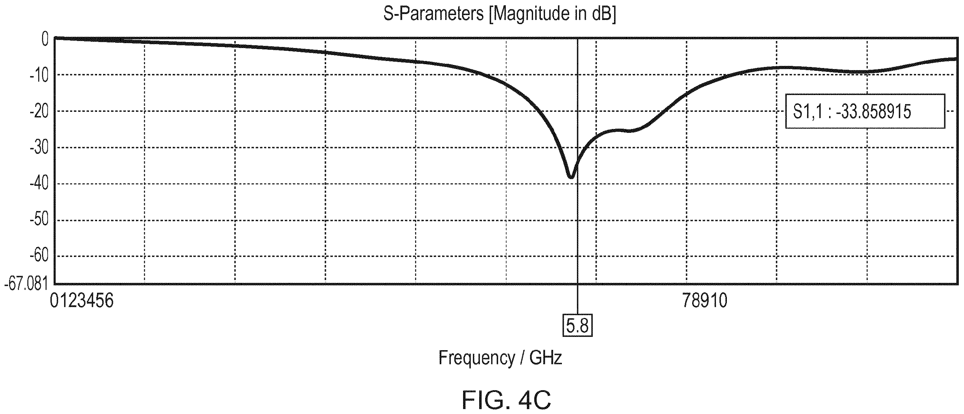

Fig. 4C shows power loss density. In this example, the power is spread over a 10 mm section which should produce a 10 mm ablation zone very quickly.

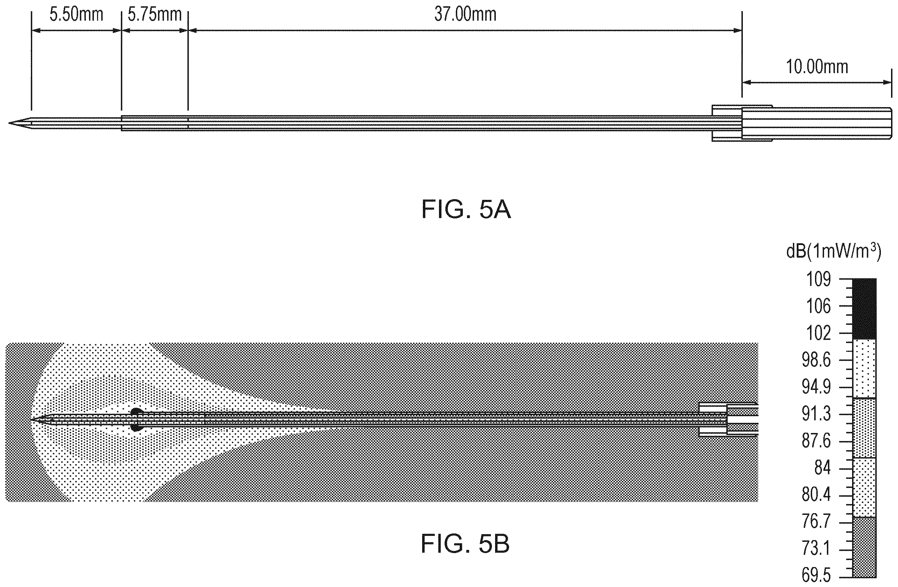

A second configuration is shown in Fig. 5A. In this example, the distal dielectric sleeve is 30% glass-filled PEEK, i.e. PEEK mixed with glass in a 70:30 ratio. The length dimensions of the three section mentioned above are altered accordingly.

Fig. 5B shows return loss for the configuration of Fig. 5A. A match at 5.8GHz is achieved with a return loss of -56.1 dB.

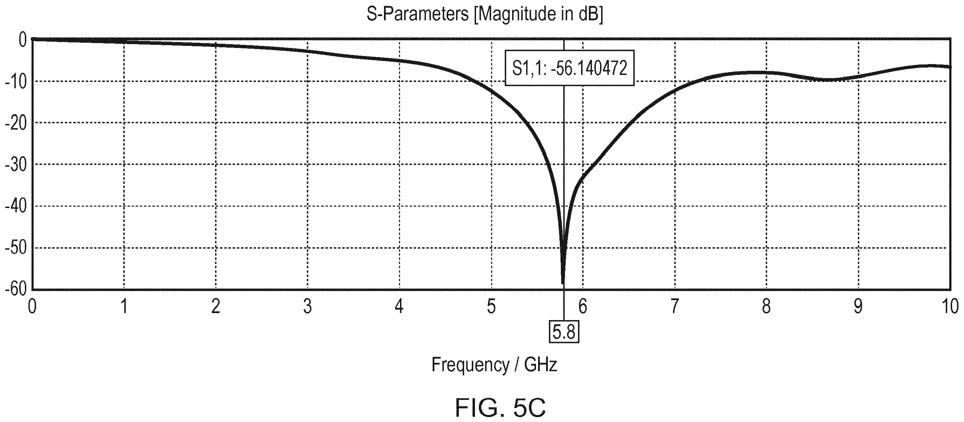

Fig. 5C shows power loss density. The profile is very similar to that shown in Fig. 4C.

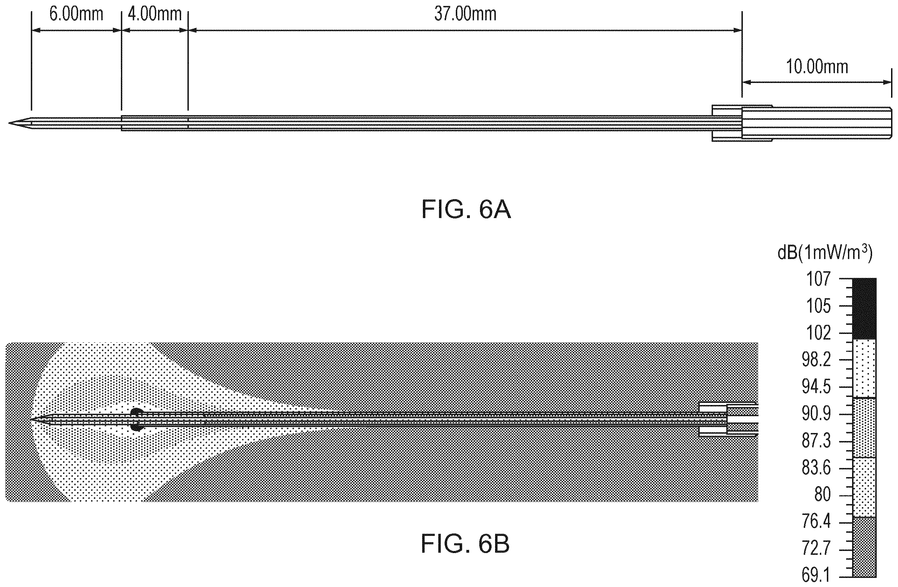

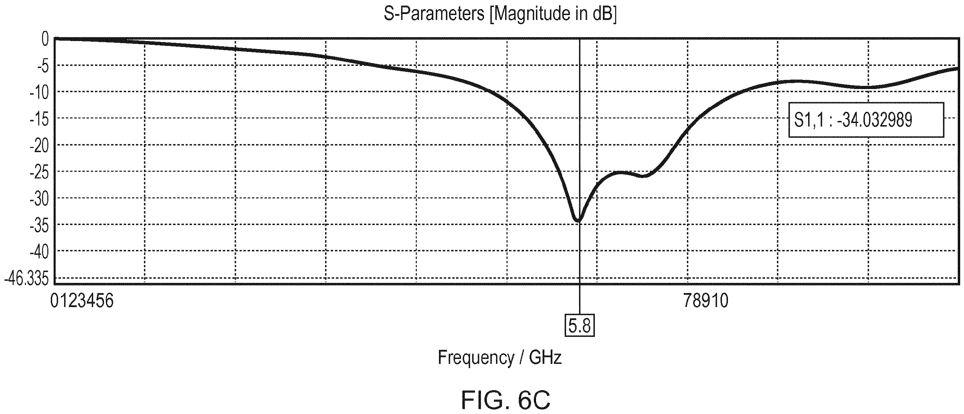

A third configuration is shown in Fig. 6A. In this example, the distal dielectric sleeve is alumina, and the length dimensions of the three section mentioned above are altered accordingly.

Fig. 5B shows return loss for the configuration of Fig. 5A. A match at 5.8GHz is achieved with a return loss of -34.0 dB.

Fig. 5C shows power loss density. The profile is very similar to that shown in Fig. 4C.

Tip materials such as alumina and glass-filled PEEK can are considered suitable for use even though they are more lossy than PEEK. The small length of the tip element means that the losses are not significant, and therefore the benefit of their better mechanical properties, i.e. for penetrating tissue, may make them suitable candidates.

In a development of the structure discussed above, it may be desirable to avoid or reduce the 'tail' of the energy profile that travels back along the outer conductor as shown in Figs. 4C, 5C and 6C. This 'tail' may cause the radiating tip portion to heat up, which may risk damaging the duodenum or causing highly undesirable collateral damage to healthy portions of the pancreas. It may thus be desirable for the instrument to exhibit a more end-fired profile, e.g. more spherical profile.

One way of achieving this may be to make the distalmost tip less sharp. Doing this may affect the impedance seen at the end of the device and therefore may require minor changes to the length of the distal dielectric sleeve. Making the distalmost tip less sharp may also make tissue insertion more difficult or more risky.

Another way of reshaping the profile is to provide one or more baluns or quarter wavelength chokes on the outer surface of the outer conductor. The position and number of the baluns will determine the resulting shape of the emitting power density profile. The more BALUNs are present, the sharper the profile will become. In one example, the baluns may be applied to the outer conductor after the instrument is assembled, e.g. by applying or affixing insulating material (e.g. annular bands of an insulator) and overlying conductive material on the outer conductor.

In use the instrument may emit microwave energy according to an energy delivery profile controlled at the generator. In one example, the microwave energy is delivered in discrete bursts or pulses. For example, microwave energy at 5.8 GHz may be delivered at 60 W using a pulsed profile having a 25% duty cycle, e.g. 1 second ON followed by 3 seconds OFF. In another example, microwave energy at 5.8GHz may be delivered at 1 kW using a pulsed profile having a 10% duty cycle, e.g. 200 microseconds ON, 1800 microseconds OFF. This latter profile could be controlled to deliver between 100 J and 3 kJ of energy over a treatment period.

## Claims

1. An electrosurgical instrument (200) comprising:
a flexible coaxial cable (202) configured to convey microwave energy;
a radiating tip portion (201) connected at a distal end of the coaxial cable and configured to receive the microwave energy, wherein the radiating tip portion is substantially rigid to permit insertion into biological tissue, and wherein the radiating tip portion comprises:
a proximal coaxial transmission line for conveying the microwave energy; and
a distal needle tip mounted at a distal end of the coaxial transmission line,
wherein the distal needle tip is configured to operate as a half wavelength transformer to deliver the microwave energy from the distal needle tip.

2. An electrosurgical instrument (200) according to claim 1, wherein the radiating tip portion comprises an intermediate coaxial transmission line between the proximal coaxial transmission line and the distal needle tip.

3. An electrosurgical instrument (200) according to claim 1 or 2, wherein the proximal coaxial transmission line comprises:
an inner conductor (204) that extends from a distal end of the flexible coaxial cable (202), the inner conductor (204) being electrically connected to an centre conductor of the flexible coaxial cable (202);
a proximal dielectric sleeve (206) mounted around the inner conductor (204); and
a outer conductor (210) mounted around the proximal dielectric (206),
wherein the distal needle tip comprises a distal dielectric sleeve (208) mounted around the inner conductor (204), and
wherein a distal portion of the outer conductor (210) overlays a proximal portion of the distal dielectric sleeve (208) .

4. An electrosurgical instrument (200) according to claim 3, wherein the inner conductor (204) has a diameter that is less than the diameter of the centre conductor of the flexible coaxial cable (202).

5. An electrosurgical instrument (200) according to claim 3 or 4, wherein the radiating tip portion is secured to the flexible coaxial cable (202) by a collar (212) mounted over a junction therebetween.

6. An electrosurgical instrument (200) according to claim 5, wherein the collar (212) is conductive and electrically connects the outer conductor (210) with an outer conductor of the flexible coaxial cable (202).

7. An electrosurgical instrument (200) according to any one of claims 3 to 6, wherein the distal dielectric sleeve (208) has a bore formed therethrough for receiving the inner conductor (204), and wherein the distal needle tip further comprises a tip element (214) mounted at a distal end of the distal dielectric sleeve (208) to close the bore.

8. An electrosurgical instrument (200) according to any one of claims 3 to 7, wherein a distal end of the distal dielectric sleeve (208) is sharpened.

9. An electrosurgical instrument (200) according to any one of claims 3 to 8, wherein distal dielectric sleeve (208) is made from a different material to the proximal dielectric sleeve (206).

10. An electrosurgical instrument (200) according to any one of claims 3 to 9, wherein the distal dielectric sleeve (208) is any of ceramic, polyether ether ketone (PEEK), glass-filled PEEK.

11. An electrosurgical instrument (200) according to any preceding claim, wherein the radiating tip portion has a length equal to or greater than 40 mm.

12. An electrosurgical instrument (200) according to any preceding claim, wherein the radiating tip portion has a maximum outer diameter equal to or less than 1.2 mm.

13. An electrosurgical instrument (200) according to any preceding claim, wherein a microwave choke or balun is fabricated on an outer surface of the proximal coaxial transmission line.

14. An electrosurgical apparatus (100) comprising:
a surgical scoping device (114) having an instrument cord (120) configured to be insertable into a patient's body, wherein the instrument cord (120) has an instrument channel (132) formed therethrough; and
an electrosurgical instrument (200) according to any preceding claim dimensioned to be insertable through the instrument channel (132).

15. An electrosurgical apparatus (100) according to claim 14, wherein the surgical scoping device (114) is an ultrasound-enabled endoscope.

## Patentansprüche

1. Elektrochirurgisches Instrument (200), das Folgendes umfasst:
ein biegsames Koaxialkabel (202), das ausgelegt ist, um Mikrowellenenergie zu transportieren;
einen Strahlungsspitzenabschnitt (201), der mit einem distalen Ende des Koaxialkabels verbunden und ausgelegt ist, um die Mikrowellenenergie aufzunehmen, wobei der Strahlungsspitzenabschnitt im Wesentlichen starr ist, um das Einführen in biologisches Gewebe zu ermöglichen, und wobei der Strahlungsspitzenabschnitt Folgendes umfasst:
eine proximale Koaxialübertragungsleitung zum Transportieren der Mikrowellenenergie; und
eine distale Nadelspitze, die an einem distalen Ende der koaxialen Übertragungsleitung angebracht ist,
wobei die distale Nadelspitze ausgelegt ist, um als Halbwellenlängenwandler betrieben zu werden, um die Mikrowellenenergie von der distalen Nadelspitze abzugeben.

2. Elektrochirurgisches Instrument (200) nach Anspruch 1, wobei der Strahlungsspitzenabschnitt eine Koaxialzwischenübertragungsleitung zwischen der proximalen Koaxialübertragungsleitung und der distalen Nadelspitze umfasst.

3. Elektrochirurgisches Instrument (200) nach Anspruch 1 oder 2, wobei die proximale Koaxialübertragungsleitung Folgendes umfasst:
einen Innenleiter (204), der sich von einem distalen Ende des biegsamen Koaxialkabels (202) erstreckt, wobei der Innenleiter (204) mit einem Mittelleiter des biegsamen Koaxialkabels (202) elektrisch verbunden ist;
eine proximale dielektrische Hülse (206), die um den Innenleiter (204) angebracht ist; und
einen Außenleiter (210), der um die proximale dielektrische Hülse (206) angebracht ist,
wobei die distale Nadelspitze eine distale dielektrische Hülse (208) umfasst, die um den Innenleiter (204) herum angebracht ist, und
wobei ein distaler Abschnitt des Außenleiters (210) einen proximalen Abschnitt der distalen dielektrischen Hülse (208) überlagert.

4. Elektrochirurgisches Instrument (200) nach Anspruch 3, wobei der Innenleiter (204) einen Durchmesser aufweist, der kleiner als der Durchmesser des Mittelleiters des biegsamen Koaxialkabels (202) ist.

5. Elektrochirurgisches Instrument (200) nach Anspruch 3 oder 4, wobei der Strahlungsspitzenabschnitt über eine Manschette (212) an dem biegsamen Koaxialkabel (202) fixiert ist, die über eine dazwischenliegende Verbindungsstelle angebracht ist.

6. Elektrochirurgisches Instrument (200) nach Anspruch 5, wobei die Manschette (212) elektrisch leitend ist und den Außenleiter (210) elektrisch mit einem Außenleiter des biegsamen Koaxialkabels (202) verbindet.

7. Elektrochirurgisches Instrument (200) nach einem der Ansprüche 3 bis 6, wobei die distale dielektrische Hülse (208) eine durch diese hindurch verlaufende Bohrung zur Aufnahme des Innenleiters (204) aufweist und wobei die distale Nadelspitze außerdem ein Spitzenelement (214) umfasst, das an einem distalen Ende der distalen dielektrischen Hülse (208) nahe der Bohrung angebracht ist.

8. Elektrochirurgisches Instrument (200) nach einem der Ansprüche 3 bis 7, wobei ein distales Ende der distalen dielektrischen Hülse (208) geschärft ist.

9. Elektrochirurgisches Instrument (200) nach einem der Ansprüche 3 bis 8, wobei die distale dielektrische Hülse (208) aus einem anderen Material besteht als die proximale dielektrische Hülse (206).

10. Elektrochirurgisches Instrument (200) nach einem der Ansprüche 3 bis 9, wobei die distale dielektrische Hülse (208) eine beliebige aus Keramik, Polyetheretherketon (PEEK), glasgefülltem PEEK ist.

11. Elektrochirurgisches Instrument (200) nach einem der vorangegangenen Ansprüche, wobei der Strahlungsspitzenabschnitt eine Länge von 40 mm oder mehr aufweist.

12. Elektrochirurgisches Instrument (200) nach einem der vorangegangenen Ansprüche, wobei der Strahlungsspitzenabschnitt einen maximalen Außendurchmesser von 1,2 mm oder weniger aufweist.

13. Elektrochirurgisches Instrument (200) nach einem der vorangegangenen Ansprüche, wobei eine Mikrowellendrossel oder ein Symmetrierglied auf einer Außenfläche der proximalen koaxialen Übertragungsleitung hergestellt ist.

14. Elektrochirurgische Vorrichtung (100), die Folgendes umfasst:
eine chirurgische Sondierungsvorrichtung (114), die ein Instrumentenkabel (120) aufweist, das ausgelegt ist, um in den Körper eines Patienten eingeführt zu werden, wobei das Instrumentenkabel (120) einen Instrumentenkanal (132) aufweist, der durch dieses hindurch ausgebildet ist; und
ein elektrochirurgisches Instrument (200) nach einem der vorangegangenen Ansprüche, das so dimensioniert ist, dass es durch den Instrumentenkanal (132) einführbar ist.

15. Elektrochirurgische Vorrichtung (100) nach Anspruch 14, wobei die chirurgische Sondierungsvorrichtung (114) ein Ultraschall-fähiges Endoskop ist.

## Revendications

1. Instrument électrochirurgical (200) comprenant :
un câble coaxial flexible (202) conçu pour transporter de l'énergie micro-onde ;
une partie formant pointe rayonnante (201) connectée à une extrémité distale du câble coaxial et conçue pour recevoir l'énergie micro-onde, dans lequel la partie formant pointe rayonnante est sensiblement rigide pour permettre une insertion dans un tissu biologique, et dans lequel la partie formant pointe rayonnante comprend :
une ligne de transmission coaxiale proximale pour transporter l'énergie micro-onde ; et
une pointe d'aiguille distale montée à une extrémité distale de la ligne de transmission coaxiale,
dans lequel la pointe d'aiguille distale est conçue pour fonctionner en tant que transformateur de demi-longueur d'onde pour délivrer l'énergie micro-onde à partir de la pointe d'aiguille distale.

2. Instrument électrochirurgical (200) selon la revendication 1, dans lequel la partie formant pointe rayonnante comprend une ligne de transmission coaxiale intermédiaire entre la ligne de transmission coaxiale proximale et la pointe d'aiguille distale.

3. Instrument électrochirurgical (200) selon la revendication 1 ou 2, dans lequel la ligne de transmission coaxiale proximale comprend :
un conducteur interne (204) qui s'étend à partir d'une extrémité distale du câble coaxial flexible (202), le conducteur interne (204) étant connecté électriquement à un conducteur central du câble coaxial flexible (202) ;
une gaine diélectrique proximale (206) montée autour du conducteur interne (204) ; et
un conducteur externe (210) monté autour du diélectrique proximal (206),
dans lequel la pointe d'aiguille distale comprend une gaine diélectrique distale (208) montée autour du conducteur interne (204), et
dans lequel une partie distale du conducteur externe (210) se superpose à une partie proximale de la gaine diélectrique distale (208).

4. Instrument électrochirurgical (200) selon la revendication 3, dans lequel le conducteur interne (204) présente un diamètre qui est inférieur au diamètre du conducteur central du câble coaxial flexible (202).

5. Instrument électrochirurgical (200) selon la revendication 3 ou 4, dans lequel la partie formant pointe rayonnante est fixée au câble coaxial flexible (202) par un collier (212) monté au-dessus d'une jonction entre eux.

6. Instrument électrochirurgical (200) selon la revendication 5, dans lequel le collier (212) est conducteur et connecte électriquement le conducteur externe (210) avec un conducteur externe du câble coaxial flexible (202).

7. Instrument électrochirurgical (200) selon l'une quelconque des revendications 3 à 6, dans lequel la gaine diélectrique distale (208) présente un alésage formé à travers elle pour recevoir le conducteur interne (204), et dans lequel la pointe d'aiguille distale comprend en outre un élément de pointe (214) monté à une extrémité distale de la gaine diélectrique distale (208) pour fermer l'alésage.

8. Instrument électrochirurgical (200) selon l'une quelconque des revendications 3 à 7, dans lequel une extrémité distale de la gaine diélectrique distale (208) est pointue.

9. Instrument électrochirurgical (200) selon l'une quelconque des revendications 3 à 8, dans lequel la gaine diélectrique distale (208) est fabriquée à partir d'un matériau différent de celui de la gaine diélectrique proximale (206).

10. Instrument électrochirurgical (200) selon l'une quelconque des revendications 3 à 9, dans lequel la gaine diélectrique distale (208) est l'une quelconque constituée de céramique, polyéther éther cétone (PEEK), PEEK armé de verre.

11. Instrument électrochirurgical (200) selon l'une quelconque des revendications précédentes, dans lequel la partie formant pointe rayonnante présente une longueur supérieure ou égale à 40 mm.

12. Instrument électrochirurgical (200) selon l'une quelconque des revendications précédentes, dans lequel la partie formant pointe rayonnante présente un diamètre externe maximal inférieur ou égal à 1,2 mm.

13. Instrument électrochirurgical (200) selon l'une quelconque des revendications précédentes, dans lequel une bobine d'arrêt micro-ondes ou un symétriseur est fabriqué(e) sur une surface externe de la ligne de transmission coaxiale proximale.

14. Appareil électrochirurgical (100) comprenant :
un dispositif d'endoscopie chirurgical (114) présentant un cordon d'instrument (120) conçu pour pouvoir être inséré dans un corps de patient, dans lequel le cordon d'instrument (120) présente un canal d'instrument (132) formé à travers lui ; et
un instrument électrochirurgical (200) selon l'une quelconque des revendications précédentes dimensionné pour pouvoir être inséré à travers le canal d'instrument (132).

15. Appareil électrochirurgical (100) selon la revendication 14, dans lequel le dispositif d'endoscopie chirurgical (114) est un endoscope activé par ultrasons.
